# EUROPEAN PATENT APPLICATION

(11) **EP 4 586 198 A1**
(43) Date of publication of application: **16.07.2025**
(21) Application number: 24150898.5
(22) Date of filing: 09.01.2024
(51) Int. Cl.: G06T 11/00

(54) **PREDICTIVE MOTION COMPENSATION**

(71) Applicant: Koninklijke Philips N.V., 5656 AG Eindhoven (NL)
(72) Inventor: BERGNER, Frank, Eindhoven (NL); KOEHLER, Thomas, Eindhoven (NL); GRASS, Michael, 5656AG Eindhoven (NL); SCHUELKE, Christophe Michael Jean, Eindhoven (NL); WILD, Sebastian, Eindhoven (NL); KABUS, Sven, Eindhoven (NL); BIPPUS, Rolf-Dieter, Eindhoven (NL)
(74) Representative: Philips Intellectual Property & Standards

(57) **Abstract**

The present invention relates to motion compensated reconstruction. A computer-implemented method is proposed to use predicted motion based on the motion trajectory obtained for the previous CT scans to perform a motion-corrected reconstruction of a subsequent CT scan within the sequence of CT scans. The CT scan reconstructed in this way contains less motion artifacts, which in turn makes its registration to the adjacent CT scans easier. Thus, the proposed approach may improve the quality in the first pass reconstruction. Compared to the existing approach that iteratively estimates motion and performs the motion compensated reconstruction, the method disclosed herein is less time-consuming and potentially more accurate process, thereby improving clinical workflow.

## Description

### FIELD OF THE INVENTION

The present invention relates to motion compensated reconstruction, and in particular relates to a computer-implemented method, to a data processing apparatus, to a computer program product, and to a computer-readable storage medium for predictive motion compensation.

### BACKGROUND OF THE INVENTION

Motion is a problem in computed tomography (CT) as the anatomy needs to stay still while the tube passes around an arc of at least 180°. Motion compensated reconstruction tries to estimate the motion and to incorporate it into the reconstruction. The estimated motion from initial images with motion artifacts may limit and/or reduce the motion compensated reconstruction quality.

### SUMMARY OF THE INVENTION

There may be a need to provide an improved motion compensated reconstruction approach.

The object of the present invention is solved by the subject-matter of the independent claims, wherein further embodiments are incorporated in the dependent claims. It should be noted that the following described aspects of the invention apply also for the computer-implemented method, the data processing apparatus, the computer program product, and the computer-readable storage medium for predictive motion compensation.

According to a first aspect of the present invention, there is provided a computer-implemented method for predictive motion compensation, the method comprising:
obtaining a data set comprising data of a sequence of computed tomography, CT, scans performed on an examination region of a subject;
selecting at least two CT scans from the sequence of CT scans,
reconstructing at least two images corresponding to the at least two different CT scans;
determining a first set of motion parameters from the at least two reconstructed images;
selecting a subsequent CT scan from the sequence of CT scans, wherein the at least two different CT scans are prior to the subsequent CT scan;
determining, based on the first determined set of motion parameters, a first estimated set of motion parameters that is likely to occur during the subsequent CT scan; and
performing a motion compensated reconstruction to reconstruct an image corresponding to the subsequent CT scan while compensating for a predicted motion described by the first estimated set of motion parameters.

Accordingly, as will be described in greater detail below, a novel motion compensated reconstruction is proposed to use predicted motion based on the motion trajectory obtained for the previous CT scans to perform a motion-corrected reconstruction of a subsequent CT scan within the sequence of CT scans. The CT scan reconstructed in this way contains less motion artifacts, which in turn makes its registration to the adjacent CT scans easier. Thus, the proposed approach may improve the quality in the first pass reconstruction. Compared to the existing approach that iteratively estimates motion and performs the motion compensated reconstruction, the method disclosed herein is less time-consuming and potentially more accurate process, thereby improving clinical workflow.

According to an exemplary embodiment of the first aspect of the present invention, the computer-implemented method further comprises:
selecting a further subsequent CT scan from the sequence of CT scans, wherein the further subsequent CT scan follows the subsequent CT scan;
determining a second set of motion parameters from the reconstructed image corresponding to the subsequent CT scan and at least one of the at least two reconstructed images corresponding to the at least two different CT scans;
determining, based on the second determined set of motion parameters, a second estimated set of motion parameters that is likely to occur during the further subsequent CT scan; and
performing a motion compensated reconstruction to reconstruct an image corresponding to the further subsequent CT scan while compensating for a predicted motion described by the second estimated set of motion parameters.

Accordingly, this first pass may be then again used to refine the estimated motion and perform a more correct motion compensated reconstruction with the real displacement, i.e., the second set of motion parameters, and not just the predicted one. This second set of motion parameters is then used for the prediction of the motion of the further subsequent CT scan.

According to an exemplary embodiment of the first aspect of the present invention, the computer-implemented method further comprises repeatedly performing steps h) to k) for one or more remaining CT scans within the sequence of CT scans.

Accordingly, the steps may be repeated to provide a motion compensated reconstruction of the entire volume.

According to an exemplary embodiment of the first aspect of the present invention, the first estimated set of motion parameters and/or the second estimated set of motion parameters comprise one or more of:
- at least one rigid body motion parameters; and
- at least one motion parameter related to a patient periodic physiological movement.

According to an exemplary embodiment of the first aspect of the present invention, the first estimated set of motion parameters and/or the second estimated set of motion parameters comprise an estimated movement velocity.

In this embodiment, the assumed physical model may be a continuation of motion with a given velocity. In this case, the velocity is estimated from the displacement and the time between both first reconstructions.

According to an exemplary embodiment of the first aspect of the present invention, the estimated movement velocity is determined via extrapolation.

From the physical model the deformation in the next time point in the trajectory may be estimated via extrapolation. In case of rigid motion, this may include also a spatial extrapolation of motion vectors into volume regions that were not covered by the initial CT scans. The prediction is used to perform the next initial reconstruction of the next CT scan directly with motion compensated reconstruction techniques.

According to an exemplary embodiment of the first aspect of the present invention, the first estimated set of motion parameters and/or the second estimated set of motion parameters comprise a change of the estimated movement velocity over time.

In this embodiment, the motion prediction may be performed on more than two previously acquired segments. In case of a non-abrupt motion this can improve the prediction accuracy of the subsequent CT scan.

According to an exemplary embodiment of the first aspect of the present invention, the sequence is generated from a single acquisition performed on the examination region of the subject along a scan trajectory by partitioning the single acquisition into a plurality of overlapping or non-overlapping segments.

According to an exemplary embodiment of the first aspect of the present invention, step g) further comprises reconstructing the image corresponding to the subsequent CT scan while compensating for a predicted motion described by the first estimated set of motion parameters and a position of the subsequent CT scan along the scan trajectory.

In some implementations, the at least two CT scans may partially overlap with each other. In this case, during motion compensated reconstruction it may be beneficial to apply portions of the first estimated set of motion parameters corresponding to the position of the subsequent CT scan along the scan trajectory, such as angle of individual projections.

According to an exemplary embodiment of the first aspect of the present invention, prior to step g), the method further comprises:
- receiving camera data acquired by a camera that monitors the examination region of the subject;
- determining, based on the camera data, motion information of the examination region of the subject;
- determining whether a difference between the first estimated set of motion parameters and the motion information obtained from the camera data is less than or equal to a predefined threshold value; and
- in response to determining that the difference between the first estimated set of motion parameters and the motion information obtained from the camera data is less than or equal to the predefined threshold value, performing step g).

In this embodiment, camera data is used to determine if the predicted motion for the subsequent CT scan as continuation of the first set of motion parameters is a good choice. This is the case when the motion continues in the same direction but it is a bad choice when a turning point in the motion is detected by the camera. In this case, the predicted motion should not be applied.

According to an exemplary embodiment of the present invention, in response to determining that the difference between the first estimated set of motion parameters and the motion information obtained from the camera data is greater than a predefined threshold value, performing a reconstruction to reconstruct an image corresponding to the subsequent CT scan, wherein the reconstruction is not motion compensated.

According to an exemplary embodiment of the first aspect of the present invention, the computer-implemented method further comprises:
- determining a severity of motion artifacts in the reconstructed image;
- in response to determining that the severity of motion artifacts in the reconstructed image exceeds a threshold value, performing a further image reconstruction, wherein the further image reconstruction is not motion compensated;
- determining a severity of motion artifacts in the further reconstructed image;
- comparing the severity of motion artifacts in the further reconstructed image with the severity of motion artifacts in the reconstructed image; and
- selecting one of the reconstructed images and the further reconstructed image with the least severe motion artifacts for registration.

In this embodiment, the severity of motion artifacts in each of the scans reconstructed at different time points may be determined, which is a predictor of the success of the registration to come. If the motion artifacts are considered too severe for a successful registration, a second reconstruction is launched. The second reconstruction is not motion compensated, and can in some rare cases be better than the motion compensated reconstruction, if the predicted motion is too bad of a guess. The severity of the motion artifacts in the second reconstruction may be determined. The reconstruction with the least severe motion artifacts is then used for the registration step.

According to a second aspect of the present invention, there is provided a data processing apparatus comprising a processor configured to perform the steps of the method according to the first aspect and any associated example.

The CT scan reconstructed in this way contains less motion artifacts, which in turn makes its registration to the adjacent CT scans easier. Thus, the proposed data processing apparatus may improve the quality in the first pass reconstruction.

According to another aspect of the present invention, there is provided a computer program product comprising instructions which, when the program is executed by a processor, cause the processor to carry out the steps of the method according to the first aspect and any associated example.

According to a first aspect of the present invention, there is provided a computer-readable storage medium having stored thereon the computer program product.

These and other aspects of the present invention will become apparent from and be elucidated with reference to the embodiments described hereinafter.

### BRIEF DESCRIPTION OF THE DRAWINGS

These and other aspects of the invention will be apparent from and elucidated further with reference to the embodiments described by way of examples in the following description and with reference to the accompanying drawings, in which
FIG. 1 schematically illustrates an exemplary imaging system.
FIG. 2 illustrates a subject, a spiral scan trajectory of an X-ray focus or focal spot of a radiation source, and a detector array.
FIG. 3 illustrates an existing motion compensated reconstruction technique.
FIG. 4 illustrates a flowchart describing the computer-implemented method for predictive motion compensation.
FIG. 5 illustrates a further flowchart describing the computer-implemented method for predictive motion compensation.
FIG. 6 illustrates a sequence of CT scans used to describe the computer-implemented method for predictive motion compensation shown in FIGS. 4 and 5.
FIG. 7 illustrates the mechanism of how image quality is increase by the proposed procedure.

It should be noted that the figures are purely diagrammatic and not drawn to scale. In the figures, elements which correspond to elements already described may have the same reference numerals. Examples, embodiments or optional features, whether indicated as non-limiting or not, are not to be understood as limiting the invention as claimed.

### DETAILED DESCRIPTION OF EMBODIMENTS

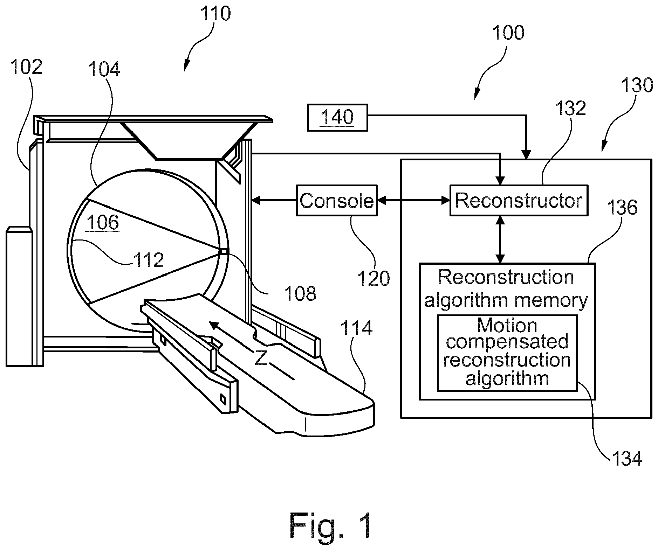

FIG. 1 schematically illustrates an exemplary imaging system 100. The exemplary imaging system 100 comprises a CT scanner 110, an operator console 120, a data processing apparatus 130, and a camera 140.

The CT scanner 110 includes a generally stationary gantry 102 and a rotating gantry 104. The rotating gantry 104 is rotatably supported by the stationary gantry 102 by a bearing or the like and rotates around an examination region 106 about a z-axis, which is the axis of rotation. A radiation source 108, such as an X-ray tube, is supported by and rotates with the rotating gantry 104, and emits X-ray radiation.

A radiation sensitive detector array 112 subtends an angular arc opposite the radiation sources 108 across the examination region 106 and detects radiation traversing the examination region 106 and generates a signal, i.e., projection data, indicative thereof. The illustrated radiation sensitive detector array 112 includes a two-dimensional (2D) array with a plurality of rows arranged with respect to each other along a direction of the z-axis.

The data processing apparatus 130 comprises a reconstructor 132 that reconstructs the signal and generates volumetric image data indicative of the examination region 106. The illustrated reconstructor 132 is configured to utilize, at least, a motion-compensated reconstruction algorithm 114 from the reconstruction algorithm memory 134.

The data processing apparatus 130 may be implemented via hardware and/or software. For example, the data processing apparatus 130 can be implemented via a processor (e.g., a central processing unit or CPU, a microprocessor, a controller, etc.) configured to execute computer executable instructions stored, encoded, embedded, etc. on computer readable medium (e.g., a memory device), which excludes a transitory medium, where executing the instructions causes the processor to perform one or more of the acts described herein and/or another act.

In the illustrated example, the reconstructor 132, the reconstruction algorithm memory 134 and the motion reconstruction algorithm 114 are shown as part of the imaging system 100. In another embodiment, the reconstructor 132, the reconstruction algorithm memory 134 and the motion reconstruction algorithm 114 are separate from the imaging system 100. In either instance, the reconstructor 132 and/or the reconstruction algorithm memory 134 and the motion reconstruction algorithm 114 can be local to or remote from the imaging system 100.

A support 114, such as a couch, supports a subject (e.g., a patient) in the examination region 106 and can be used to position the subject with respect to x, y, and/or z axes before, during and/or after scanning. A computing system serves as the operator console 120, and includes an output device such as a display configured to display the reconstructed images and an input device such as a keyboard, mouse, and/or the like. Software resident on the console 120 allows the operator to control the operation of the system 100, e.g., selecting a reconstruction parameter, etc.

A camera 140 may be placed in the room or may be attached to the stationary part 102 of the gantry. The camera 140 may be coupled to the data processing apparatus 130 via a wired or wireless connection. In some examples, the camera may be a camera that captures a two-dimensional image. In some examples, the camera 140 may be a range camera that is configured to acquire three-dimensional (3D) image data by exposure of some to non-ionizing radiation or sound. The 3D image data is an array of pixels each pixel having a position in said array and a value. Each pixel position corresponds to a position of a point on the subject's surface and the value is directly related to or can be expressed in terms of a distance between camera's sensor and said subject surface point. Pixel values vary with sensor-object distance. Point cloud representation of the 3D image data set is also envisaged. In other words the 3D image data "follows" or describes the outer surface or perimeter of the subject in 3D space. It will be appreciated that the camera 140 may be arranged at any suitable position. In some examples, the camera 140 may be arranged on the ceiling, on the overhead carriage, on the wall, or on the wall-stand. In some implementations, the camera 140 may be positioned to capture essentially the entirety of an examination region or at least that portion of the examination region where the subject can be expected to reside during the image acquisition.

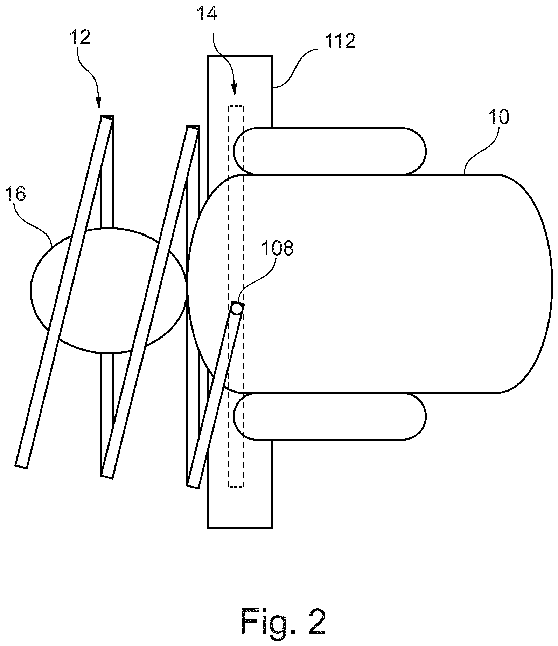

FIG. 2 illustrates a subject 10, a spiral scan trajectory 12 of an X-ray focus or focal spot of the radiation source 108, and the detector array 112. The rotating gantry 104 moves relative to the subject 10 in the z-direction and the detector array 110 collects data for different slice positions, such as slice position 14 shown in FIG. 2.

In this example, subject motion during scanning may lead to image artifacts such as blurring and/or other image artifacts in the reconstructed volumetric image data. Motion compensated CT reconstruction is a known tool to virtually improve the temporal resolution of computed tomography devices. The most basic approach is to first reconstruct two initial images at two distinct time points. Then the displacement between the voxels in both images is estimated using registration techniques. Finally, the gained knowledge about the displacement is fed into a reconstruction method that compensates for the given deformation, and then yields an image ideally without motion artifacts.

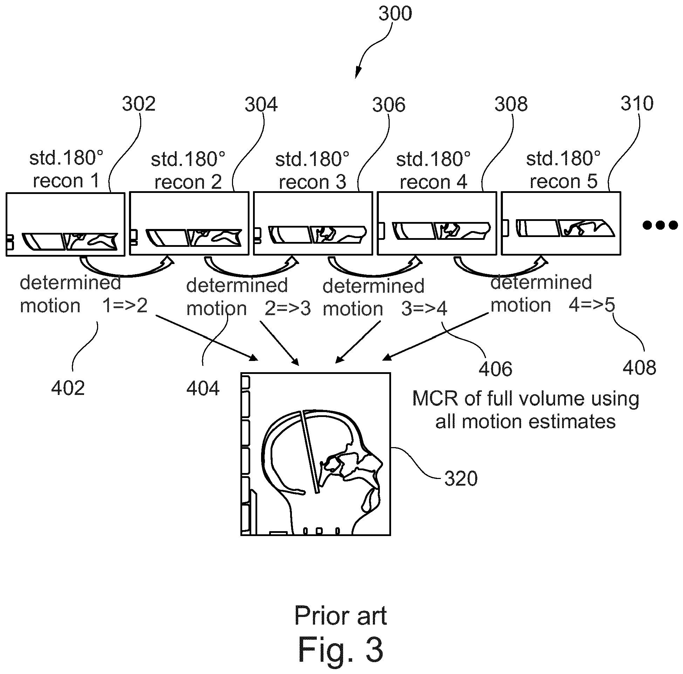

An exemplary processing scheme is illustrated in FIG. 3. In this illustrated example, a helical trajectory is scanned with the imaging system 100 shown in FIG. 1 to obtain a data set comprising data of a sequence of CT scans performed on an examination region 16, i.e., head, of the subject. The sequence is generated from a single acquisition performed on the examination region 16 of the subject along a scan trajectory by partitioning the single acquisition into a plurality of overlapping or non-overlapping segments. Data is reconstructed using 180° and fan-angle ranges in increments of, for example, 180°. At a first step, two initial images at two distinct time points are reconstructed to form two reconstructed images. In the illustrated example, the images are formed by partitioning the single acquisition into a plurality of overlapping or non-overlapping segments. Therefore, the images may also be referred to as image segments. In the illustrated example, a first image segment 302 and a second image segment 304 are reconstructed to form two reconstructed images. At a second step, the displacement between the voxels in both reconstructed images is estimated e.g., using registration techniques to determine a set of motion parameters 402. At a third step, the set of motion parameters 402 is fed into a reconstruction method that compensates for the given deformation, and then yields an image ideally without motion artifacts. Such procedure may be repeated for each subsequent image segment. A final reconstruction that provides a motion compensated reconstruction of the entire volume 320.

However, the approach shown in FIG. 3 may heavily depend on the quality of the initial reconstructions. If motion artifact levels are too high, the registration method between the two volumes can fail, e.g. caused by non-matching structures. One way to handle this is to iteratively estimate motion and perform the motion compensated reconstruction. However, this is a time-consuming and potentially erroneous process, hampering clinical workflow.

In order to address one or more of the above-described problems, a computer-implemented method and a data processing apparatus are proposed for predictive motion compensation. The computer-implemented method and the data processing apparatus as disclosed herein may compensate for voluntary, involuntary, periodic, no-periodic, and/or other motion. The computer-implemented method and the data processing apparatus as disclosed herein may also be applied to a continuous circular acquisition which is reconstructed as a sliding window reconstruction. As a specific case the computer-implemented method and the data processing apparatus as disclosed herein may be applied to data acquired during CT guided interventions (e.g., needle-based procedures). In general, with the proposed approach described herein, it is possible to recursively use previous motion estimations in combination with a physical model to predict present motion and use it for reconstructing the next time-step and part of the trajectory. Accordingly, the quality of the first pass reconstruction may be improved. The proposed computer-implemented method may be embodied as, or in, the motion compensated reconstruction algorithm 114 shown in FIG. 1. The proposed data processing apparatus may be embodied as, or in, the data processing apparatus 130 shown in FIG. 1.

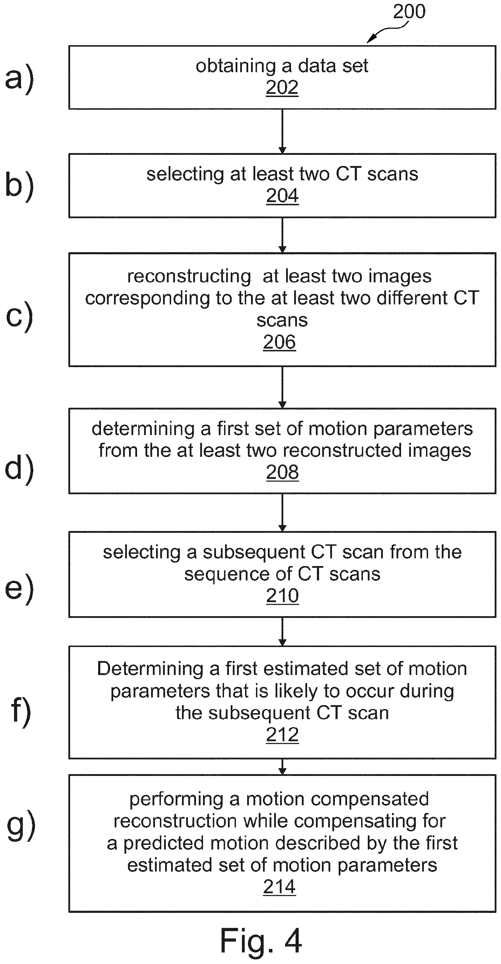

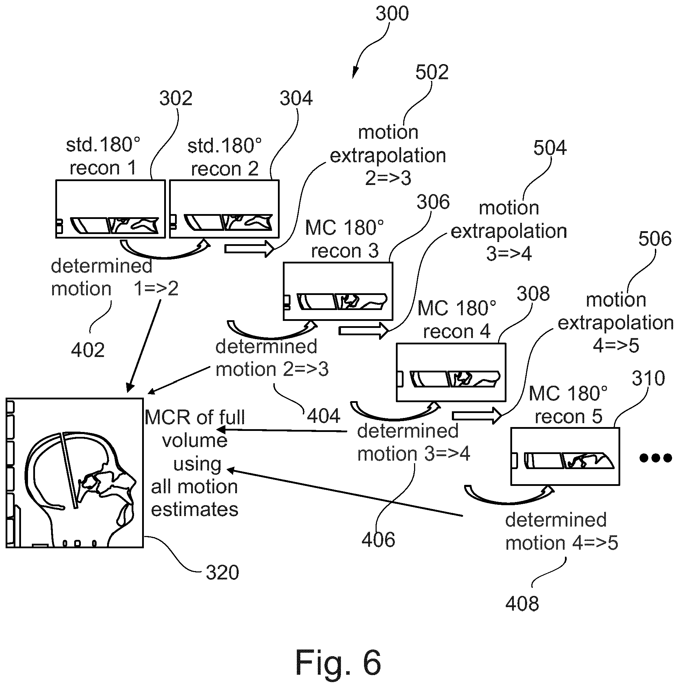

FIG. 4 illustrates a flowchart describing the computer-implemented method 200 for predictive motion compensation. The method 200 is now described in greater detail in connection with FIG. 6.

At block 202, i.e., step a), the method 200 comprises the step of obtaining a data set comprising data of a sequence of CT scans performed on an examination region of the subject. For example, the CT scanner 110 shown in FIG. 1 may perform a scan on an examination region 16 of the subject 10 along a scan trajectory 12 shown in FIG. 2. In this example, the scan is a single acquisition performed on the examination region 16 of the subject along the scan trajectory 12, and the sequence of the CT scans may be generated from the single acquisition by partitioning the single acquisition into a plurality of overlapping or non-overlapping segments. For example, the single acquisition may be partitioned using 180° plus fan-angle ranges in increments of, for example, 180°. For sufficiently low pitch, this results in a sequence of overlapping image segments along the rotation axis. The mean temporal difference between the image segments is given by the time needed to cover these 180°. For example, FIG. 3 and 6 illustrate a sequence of CT scans 300 including five exemplary image segments 302, 304, 306, 308, and 310.

Turning back to FIG. 4, at block 204, i.e., step b), the method 200 further comprises the step of selecting at least two CT scans from the sequence of CT scans. For example, as shown in FIG. 6, a first image segment 302 and a second image segment 304 are selected from the sequence of CT scans 300.

Turning back to FIG. 4, at block 206, i.e., step c), the method 200 further comprises the step of reconstructing at least two images corresponding to the at least two different CT scans. For example, as shown in FIG. 6, the first image segment 302 and the second image segment 304 are reconstructed by the reconstructor 132 shown in FIG. 1.

Turning back to FIG. 4, at block 208, i.e., step d), the method 200 further comprises the step of determining a first set of motion parameters from the at least two reconstructed images. For example, as shown in FIG. 6, the first set of motion parameters 402 is determined from the first reconstructed image segment 302 and the second reconstructed image segment 304. This may be done by analyzing the displacement between the voxels in both reconstructed images using registration techniques. In some examples, the set of motion parameters may comprise at least one rigid body motion parameter related to voluntary motion or involuntary motion (e.g., coughing, hiccups, or bowel motion). In some examples, the set of motion parameters may comprise at least one motion parameter related to a patient periodic physiological movement (e.g., cardiac cycle and/or the respiratory cycle).

Turning back to FIG. 4, at block 210, i.e., step e), the method 200 further comprises the step of selecting a subsequent CT scan from the sequence of CT scans. The at least two different CT scans are prior to the subsequent CT scan. For example, as shown in FIG. 6, a third image segment 306 is selected from the sequence of CT scans 300.

Turning back to FIG. 4, at block 212, i.e., step f), the method 200 further comprises the step of determining, based on the first determined set of motion parameters, a first estimated set of motion parameters, e.g., extrapolated motion parameters shown in FIG. 6, that is likely to occur during the subsequent CT scan. For example, as shown in FIG. 6, the first estimated set of motion parameters 502 is determined based on the first determined set of motion parameters 402. The first estimated set of motion parameters 502 describes a motion that is likely to occur during the subsequent CT scan, i.e., the third image segment 306.

The first estimated set of motion parameters 502 may be determined in various approaches. In one example, physics-based methods may be used to predict the first estimated set of motion parameters 502. Such method is based on a physical model with its parameters estimated from the first determined set of motion parameters 402. In the simplest case, the assumed physical model may be only a continuation of motion with a given velocity. In this case here, the first set of motion parameters 402 may comprise a movement velocity that is determined from the displacement and the time between the at least two reconstructed images 302 and 304. From the physical model, the motion in the subsequent CT scan, i.e., the third image segment 306, may be estimated via extrapolation. If the motion is a rigid patient motion, this may also include a spatial extrapolation of the first set of motion parameters into volume regions that were not covered by the initial segments. In some examples, the first set of motion parameters may further comprise a change of the estimated movement velocity overtime, i.e., acceleration. The change of the estimated movement velocity over time may be determined based on more than two image segments. In case of a non-abrupt motion this can improve the prediction accuracy of the subsequent segment. The estimated acceleration is then used together with the estimated velocity to predict the next displacements in the subsequent CT scan.

Turning back to FIG. 4, at block 214, i.e., step g), the method 200 further comprise the step of performing a motion compensated reconstruction to reconstruct an image corresponding to the subsequent CT scan while compensating for a predicted motion described by the first estimated set of motion parameters.

For example, as shown in FIG. 6, a motion compensated reconstruction is performed to reconstruct an image corresponding to the subsequent CT scan, i.e., the third image segment 306, while compensating for a predicted motion described by the first estimated set of motion parameters 502.

The above-described process may go on with processing the fourth image segment 308 by selecting second image segment 304 and the third image segment 306 as the at least two segments and the fourth image segment 308 being the subsequent one.

In some implementations, step g) may further comprise the step of reconstructing the image corresponding to the subsequent CT scan while compensating for a predicted motion described by the first estimated set of motion parameters and a position of the subsequent CT scan along the scan trajectory. For example, the first estimated set of motion parameters may be used to compensate for motion on a sub-segment level. As an example, as shown in FIG. 6, the first image segment 302 and the second image segment 304 may partially overlap with each other. Therefore, the first image segment 302 and the second image segment 304 may be reconstructed e.g., from ranges [0°, 180°] and [120°, 300°]. If *u* is the estimated motion for a certain spatial position, *u* describes the motion between the first image segment 302 centered around 90° and the second image segment 304 centered around 210°. However, projections acquired at e.g. an angle of 150° were affected by *u*/2 rather than *u.* Accordingly, during motion compensated reconstruction, it may be beneficial to apply portions of *u* according to the corresponding angle of individual projections, i.e., a position of the subsequent CT scan along the scan trajectory.

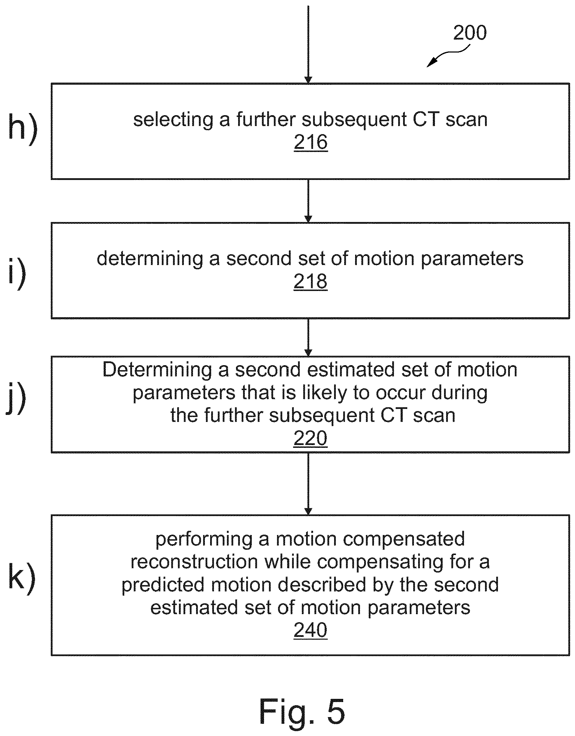

In some implementations, prior to step g), the method 200 may further comprise the steps of receiving camera data acquired by a camera that monitors the examination region of the subject, determining, based on the camera data, motion information of the examination region of the subject, and determining whether a difference between the first estimated set of motion parameters and the motion information obtained from the camera data is less than or equal to a predefined threshold value. In response to determining that the difference between the first estimated set of motion parameters and the motion information obtained from the camera data is less than or equal to the predefined threshold value, the method 200 may comprise the step of performing step g). On the other hand, in response to determining that the difference between the first estimated set of motion parameters and the motion information obtained from the camera data is greater than a predefined threshold value, the method 200 may comprise the step of performing a reconstruction to reconstruct an image corresponding to the subsequent CT scan. The reconstruction is not motion compensated. For example, as shown in FIG. 1, camera data may be captured by the camera 140. As shown in FIG. 5, the camera data is used to determine if the predicted motion 502 for the subsequent CT scan, i.e., the third image segment 306, as continuation of the first set of motion parameters 402 is a good choice. This is the case when the motion continues in the same direction, but it is a bad choice when a turning point in the motion is detected by the camera 140. In this case, the predicted motion should not be applied.

This first pass may be then again used to refine the estimated motion and perform a more correct motion compensated reconstruction with the real displacement and not just the predicted one. This process is shown in FIG. 5.

As shown in FIG. 5, at block 216, i.e., step h), the method 200 may further comprise the step of selecting a further subsequent CT scan. For example, as shown in FIG. 6, a fourth image segment 308 may be selected from the sequence of CT scans.

Turning back to FIG. 5, at block 218, i.e., step i), the method 200 may further comprise the step of determining a second set of motion parameters from the reconstructed image corresponding to the subsequent CT scan and at least one of the at least two reconstructed images corresponding to the at least two different CT scans. For example, as shown in FIG. 6, a second set of motion parameters 404 is determined from the third reconstructed image segment 306 and at least one of the first reconstructed image segment 302 and the second reconstructed image segment 304. The second set of motion parameters may comprise a movement velocity and optionally an acceleration, i.e. the change of movement velocity over time.

Turning back to FIG. 5, at block 220, i.e., step j), the method 200 may further comprise the step of determining, based on the second determined set of motion parameters, a second estimated set of motion parameters that is likely to occur during the further subsequent CT scan. For example, as shown in FIG. 6, a second estimated set of motion parameters 504 is determined based on the second determined set of motion parameters 404. As described above, the second estimated set of motion parameters 504 may be determined using a physical model. In one example, the assumed physical model may be only a continuation of motion with a given velocity. In some examples, the second estimated set of motion parameters may further comprise a change of the estimated movement velocity over time, i.e., acceleration.

Turning back to FIG. 5, at block 222, i.e., step k), the method 200 may further comprise the step of performing a motion compensated reconstruction to reconstruct an image corresponding to the further subsequent CT scan while compensating for a predicted motion described by the second estimated set of motion parameters. For example, as shown in FIG. 6, a motion compensated reconstruction may be performed by the reconstructor 132 shown in FIG. 1 to reconstruct the fourth image segment 308 while compensating for a predicted motion described by the second estimated set of motion parameters 504.

In some implementations, the method 200 may further comprise repeatedly performing steps h) to k) for one or more remaining CT scans within the sequence of CT scans. For example, as shown in FIG. 6, steps h) to k) may be repeatedly performed for one or more remaining image segments, such as image segment 310. A final reconstruction is to a motion compensated reconstruction of the entire volume 320.

Optionally, the method 200 may further comprise the steps of determining a severity of motion artifacts in the reconstructed image, in response to determining that the severity of motion artifacts in the reconstructed image exceeds a threshold value, performing a further image reconstruction, wherein the further image reconstruction is not motion compensated, determining a severity of motion artifacts in the further reconstructed image, comparing the severity of motion artifacts in the further reconstructed image with the severity of motion artifacts in the reconstructed image, and selecting one of the reconstructed image and the further reconstructed image with the least severe motion artifacts for registration. For example, a neural network may be designed for that purpose and returns an indicator of the severity of motion artifacts in each of the scans reconstructed at different time points. The indicator may be a predictor of the success of the registration to come. If the motion artifacts are considered too severe for a successful registration, a second reconstruction is launched. The second reconstruction is not motion compensated, and can in some rare cases be better than the motion compensated reconstruction, if the extrapolated motion is too bad of a guess. The neural network then rates the severity of the motion artifacts in the second reconstruction, and the reconstruction with the least severe motion artifacts is then used for the registration step. For the motion artifact severity rules on the image sharpness could be used as motion typically blurs the images. The order of the two reconstructions can also be switched: the first reconstruction does not correct for motion, and a second reconstruction with compensation of the extrapolated trajectory is only performed if the quality of the first reconstruction is too bad. After that, the second reconstruction is rated as well and the better of the two reconstructions is used for the subsequent registration.

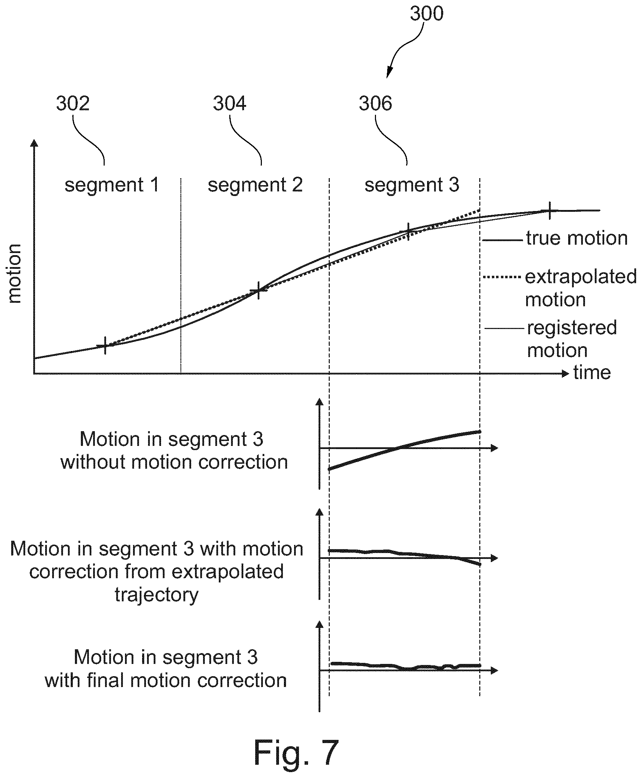

FIG. 7 illustrates the mechanism of how image quality is improved by the proposed procedure. In the illustrated example, the time series data of true motion, extrapolated motion, and registered motion is illustrated for different image segments, such as the first image segment 302, the second image segment 304, and third image segment 306. The individually reconstructed segments contain motion that can corrupt them enough to make registration between adjacent segments difficult. The proposed method uses extrapolation of the motion trajectory obtained for the previous segments to perform a motion-corrected reconstruction of the next segment. If the extrapolation is good enough, the segment reconstructed in this way contains less motion artifacts, which in turn makes its registration to the adjacent segments easier. For illustration, the interpolation and extrapolation shown here are only linear. It will be appreciated that in some other implementations, the interpolation and extrapolation may be nonlinear.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A computer-implemented method (200) for predictive motion compensation, the method comprising:
obtaining (202) a data set comprising data of a sequence of computed tomography, CT, scans performed on an examination region of the subject;
selecting (204) at least two CT scans from the sequence of CT scans;
reconstructing (206) at least two images corresponding to the at least two selected CT scans;
determining (208) a first set of motion parameters from the at least two reconstructed images;
selecting (210) a subsequent CT scan from the sequence of CT scans, wherein the at least two selected CT scans are prior to the subsequent CT scan;
determining (212), based on the first determined set of motion parameters, a first estimated set of motion parameters that is likely to occur during the subsequent CT scan; and
performing (214) a motion compensated reconstruction to reconstruct an image corresponding to the subsequent CT scan while compensating for a predicted motion described by the first estimated set of motion parameters.

2. The computer-implemented method according to claim 1, further comprising:
selecting (216) a further subsequent CT scan from the sequence of CT scans, wherein the further subsequent CT scan follows the subsequent CT scan;
determining (218) a second set of motion parameters from the reconstructed image corresponding to the subsequent CT scan and at least one of the at least two reconstructed images corresponding to the at least two selected CT scans;
determining (220), based on the second determined set of motion parameters, a second estimated set of motion parameters that is likely to occur during the further subsequent CT scan; and
performing (222) a motion compensated reconstruction to reconstruct an image corresponding to the further subsequent CT scan while compensating for a predicted motion described by the second estimated set of motion parameters.

3. The computer-implemented method according to claim 2, further comprising:
repeatedly performing steps h) to k) for one or more remaining CT scans within the sequence of CT scans.

4. The computer-implemented method according to any one of claims 1 to 3,
wherein the first estimated set of motion parameters and/or the second estimated set of motion parameters comprise one or more of:
- at least one rigid body motion parameters; and
- at least one motion parameter related to a patient periodic physiological movement.

5. The computer-implemented method according to any one of the preceding claims,
wherein the first estimated set of motion parameters and/or the second estimated set of motion parameters comprise an estimated movement velocity.

6. The computer-implemented method according to claim 5,
wherein the estimated movement velocity is determined via extrapolation.

7. The computer-implemented method according to claim 5 or 6,
wherein the first estimated set of motion parameters and/or the second estimated set of motion parameters comprise a change of the estimated movement velocity over time.

8. The computer-implemented method according to any one of the preceding claims,
wherein the sequence is generated from a single acquisition performed on the examination region of the subject along a scan trajectory by partitioning the single acquisition into a plurality of overlapping or non-overlapping segments.

9. The computer-implemented method according to claim 8,
wherein step g) further comprises reconstructing the image corresponding to the subsequent CT scan while compensating for a predicted motion described by the first estimated set of motion parameters and a position of the subsequent CT scan along the scan trajectory.

10. The computer-implemented method according to any one of the preceding claims,
wherein prior to step g), the method further comprises:
- receiving camera data acquired by a camera that monitors the examination region of the subject;
- determining, based on the camera data, motion information of the examination region of the subject;
- determining whether a difference between the first estimated set of motion parameters and the motion information obtained from the camera data is less than or equal to a predefined threshold value; and
- in response to determining that the difference between the first estimated set of motion parameters and the motion information obtained from the camera data is less than or equal to the predefined threshold value, performing step g).

11. The computer-implemented method according to claim 10,
wherein in response to determining that the difference between the first estimated set of motion parameters and the motion information obtained from the camera data is greater than a predefined threshold value, performing a reconstruction to reconstruct an image corresponding to the subsequent CT scan, wherein the reconstruction is not motion compensated.

12. The computer-implemented method according to any one of the preceding claims, further comprising:
- determining a severity of motion artifacts in the reconstructed image;
- in response to determining that the severity of motion artifacts in the reconstructed image exceeds a threshold value, performing a further image reconstruction, wherein the further image reconstruction is not motion compensated;
- determining a severity of motion artifacts in the further reconstructed image;
- comparing the severity of motion artifacts in the further reconstructed image with the severity of motion artifacts in the reconstructed image; and
- selecting one of the reconstructed image and the further reconstructed image with the least severe motion artifacts for registration.

13. A data processing apparatus (130) comprising a processor configured to perform the steps of the method according to any one of the preceding claims.

14. A computer program product comprising instructions which, when the program is executed by a processor, cause the processor to carry out the steps of the method according to any one of claims 1 to 12.

15. A computer-readable storage medium having stored thereon the computer program product of claim 14.
